# EUROPEAN PATENT SPECIFICATION

(11) **EP 2 571 494 B1**
(45) Date of publication and mention of the grant of the patent: **11.01.2017**
(21) Application number: 11714599.5
(22) Date of filing: 07.04.2011
(51) Int. Cl.: A61K 38/00, A61K 9/19, A61L 24/10, A61L 26/00, A61P 7/04

(54) **HEMOSTATIC COMPOSITIONS**
HÄMOSTATISCHE ZUBEREITUNGEN
COMPOSITIONS HÉMOSTATIQUES

(30) Priority: 20.05.2010 GB 201008404
(43) Date of publication of application: 27.03.2013
(73) Proprietor: FUJIFILM Corporation, Tokyo 106-8620 (JP)
(72) Inventor: VAN DONGEN, Elisabeth, NL-5047 TK Tilburg (NL)
(74) Representative: Hoffmann Eitle
(86) International application number: PCT/GB2011/050689
(87) International publication number: WO 2011/144916

(56) References cited:
- EP-A1- 1 961 414
- WO-A1-2008/103041
- WO-A2-2010/038059
- GB-A- 2 414 021
- US-A1- 2008 085 316

## Description

### FIELD OF INVENTION

The present invention is directed to cross-linked gelatin compositions for medical use as a hemostatic agent. The present invention is further directed to the preparation of such compositions. These compositions are useful as hemostatic agents in a variety of medical applications, including vascular plug type devices, wound closure devices, dressings for use to treat incisions, seeping wounds, and the like.

### BACKGROUND OF THE INVENTION

Hemostatic agents have a wide range of use from immediate trauma management to use in surgical procedures. These materials help control bleeding from various types of traumas such as open skin wounds and spleen and kidney injuries.

Biodegradable hemostatic agents produced from naturally derived sources have been available for decades. Examples of these are Gelfoam® manufactured by Up-John and disclosed in US Patent No. 2,465,357; Avitene® manufactured by Acecon and disclosed in US Patent No. 3,742,955 and Surgicell® manufactured by Johnson and Johnson and disclosed in US Patent No. 3,364,200. These biodegradable hemostatic agents are primarily formulated using collagen, gelatin and cellulose. Among these materials those made of collagen are the only types known to interact with platelets and activate the clotting cascades to stop bleeding, in a similar way to physiological hemostasis.

Platelets play an important role in the initial stages of hemostasis. Thus, following injury platelets adhere to exposed subendothelial connective tissue; additional platelets then bind to the layer of adhered platelets and form a plug which acts as a barrier to further bleeding. The adhered platelets also release clot-promoting factors and accelerate blood coagulation.

Collagen is a major component of connective tissue and it is well-known that platelets interact with collagen fiber by adhesion and aggregation. In other words, collagen is important in the initiation of hemostasis.

Further improvements on collagen derived hemostatic agents have increased their speed of action. Thus, US Patent No. 4,215,200 describes chemical modification of collagen fibers to increase the electrostatic charge to a high positive charge at physiological pH. However these treatments introduce the risk of unwanted chemical residues.

Related art is disclosed in GB 2414021, US 2008/085316, EP 1961414, WO 2010/038059, WO 2008/103041.

Moreover the use of animal-source collagen in hemostatic agents has given rise to safety issues, such as concern over potential immunogenic, e.g., antigenic and allergenic responses. The inability to completely characterize, purify, or reproduce animal-source collagen or gelatin mixtures used currently is of ongoing concern in the pharmaceutical and medical communities. Additional safety concerns exist with respect to bacterial contamination and endotoxin loads resulting from the extraction and purification processes. Recombinantly produced collagen-like peptides or gelatins are a solution to these safety concerns. The use of recombinant collagen-like peptides is disclosed in International Patent Application WO2004078120, however this publication only teaches that material made from recombinant collagen fibrils resembles the microstructure of commercially available hemostatic sponge. International Patent Application WO200009018 teaches the use of a recombinant collagen III in a hemostatis agent. However the recombinant collagen described is also fibrillar in nature due to recombinant production in the presence of a prolyl-4-hydroxylase. The homo- or heterotrimeric configuration mimics native collagen. However homo- or heterotrimeric collagen-like proteins are incompatible with the most efficient recombinant production systems where the proteins are secreted by a microorganism (especially the preferred *Pichia* host system). These secretion based systems have many advantages, for example the yield of the secreted recombinant proteins are significantly higher. Another advantage is that downstream processing to purify secreted recombinant proteins is much less elaborate since it does not require disruption of the host-cells to obtain the recombinant product. Therefore, there is need for hemostatic agents based on recombinant collagen-like or gelatin proteins which are effective without requiring chemical modification and easier to produce than the currently known collagen derived agents.

### GENERAL DEFINITIONS

The term "comprising" is to be interpreted as specifying the presence of the stated parts, steps or components, but does not exclude the presence of one or more additional parts, steps or components.

Reference to an element by the indefinite article "a" or "an" does not exclude the possibility that more than one of the element is present, unless the context clearly requires that there be one and only one of the elements. The indefinite article "a" or "an" thus usually means "at least one".

The terms "protein" or "polypeptide" or "peptide" are used interchangeably and refer to molecules consisting of a chain of amino acids, without reference to a specific mode of action, size, three-dimensional structure or origin.

"Gelatin" as used herein refers to any gelatin, whether extracted by traditional methods or recombinant or biosynthetic in origin, or to any molecule having at least one structural and/or functional characteristic of gelatin. Gelatin is currently obtained by extraction from collagen derived from animal (e.g., bovine, porcine, rodent, chicken, equine, piscine) sources, e.g., bones and tissues. The term encompasses both the composition of more than one polypeptide included in a gelatin product, as well as an individual polypeptide contributing to the gelatin material. Thus, the term recombinant gelatin as used in reference to the present invention encompasses both a recombinant gelatin material comprising gelatin polypeptides, as well as an individual gelatin polypeptide. Polypeptides from which gelatin can be derived are polypeptides such as collagens, procollagens, and other polypeptides having at least one structural and/or functional characteristic of collagen. Such a polypeptide could include a single collagen chain, or a collagen homotrimer or heterotrimer, or any fragments, derivatives, oligomers, polymers, or subunits thereof, containing at least one collagenous domain (Gly-X-Y region). The term specifically contemplates engineered sequences not found in nature, such as altered collagen sequences, e.g. a sequence that is altered, through deletions, additions, substitutions, or other changes, from a naturally occurring collagen sequence. Such sequences may be obtained from suitable altered collagen polynucleotide constructs, etc.

A "cross-linking agent" as described herein refers to a composition comprising a cross-linker. "Cross-linker" as used herein refers to a reactive chemical compound that is able to introduce covalent intra- and inter- molecular bridges in organic molecules.

### DETAILED DESCRIPTION OF THE INVENTION

The present invention now will be described more fully. However this invention may be embodied in many different forms and should not be construed as limited to the specific embodiments as set forth herein.

The present invention provides a composition comprising a cross-linked gelatin with an isoelectric point of at least 7, wherein the gelatin is a recombinant gelatin which comprises at least one RGD motif and the gelatin is cross-linked using a carbodiimide, for use as a hemostatic agent.

Preferably the gelatin which is to be cross-linked has an isoelectric point of at least 7, more preferably of at least 8, especially of at least 9, more especially of at least 10 and particularly of at least 11 before cross-linking.

Preferably after cross-linking the cross-linked gelatin has an iso-electric point of at least 8, more preferably of at least 9, especially of at least 10, and more especially of at least 11.

The isoelectric point of the gelatin and cross-linked gelatin is calculated based on the amino acid composition of the gelatins.

Preferably the gelatin which is to be cross-linked which is a recombinant gelatin is as in EP 0926543, EP 1014176 and WO 01/34646, and also specifically the examples of EP 0926543 and EP 1014176 which describe preferred recombinant gelatins and their production methods, using methylotrophic yeasts, in particular *Picha pastoris.*

An advantage of recombinant gelatins is that the amino acid sequence can be manipulated to create certain characteristics. Examples of characteristics that can be manipulated are (i) the isoelectric point and charge density of the recombinant gelatin (for example charged amino acids, such as asparagine (Asn), aspartic acid (Asd), glutamine (Gln), glutamic acid (Glu) or lysine (Lys) can be introduced or left out) (ii) the glycosylation pattern (for example the absence of threonine and/or serine amino acids in certain positions results in the absence of glycosylation), (iii) the size of the recombinant gelatin, (iv) the amount of cross-linkable amino acids (for example the amount of lysines), (v) the biodegradability can be amended by the presence or absence of cleavage sites for metalloproteinases.

In a preferred embodiment the gelatin which is to be cross-linked comprises a ratio between the total of arginine and lysine and the total of glutamine and asparagine amino acid residues that is at least 1.0 and more preferably at least 1.1. In a more preferred embodiment, the invention provides a hemostatic composition wherein the gelatin to be cross-linked, preferably a recombinant gelatin, comprises at least 0.40 mmol/g lysine residues before cross-linking, more preferably at least 0.60mmol/g and especially at least 0.80 mmol/g.

Clearly if more cross-linkable groups are available, the amount of crosslinks can in principle be higher if compared to a situation in which less cross-linkable groups are present. Also, the amount of cross-linked groups determine the biodegradability of the cross-linked gelatin. When a recombinant gelatin is used it is possible to control the amount of cross-linkable groups and thus the biodegradability of the cross-linked gelatin can be manipulated.

The optimum degree of cross-linking depends on the intended use of the hemostatic agent and intended method of application. The minimum degree of cross-linking should result in a hemostatic agent that does not dissolve after application.

In another preferred embodiment the recombinant gelatin of the composition is a non-gelling recombinant gelatin with a Bloom strength of below 10g.

It is also preferred the recombinant gelatin is free of hydroxyproline and hydroxylysine residues. Hydroxylated residues, such as these, allow the formation of intra and extra molecular hydrogen bridges and give rise to the homo or heterotrimeric collagen structures, which are incompatible with the preferred production method via extracellular secretion by a yeast, especially *Pichia pastoris.* Examples of recombinant gelatins compatible with extracellular secretion are disclosed in EP 0926543, EP 1014176 and WO01/34646.

In a further preferred embodiment functionalized recombinant gelatins for enhanced cell binding and/or with minimal immunogenicity such as, for example, those disclosed in EP 1608681 and EP 1368056 can be cross-linked to form the hemostatic compositions of the invention.

In another preferred embodiment the recombinant gelatins used in the compositions of the present invention have a molecular weight of at least 25 kDa, more preferably of at least 35 kDa and most preferably of at least 50 kDa.

The recombinant gelatin is a gelatin enriched in RGD motifs. The term 'RGD-enriched gelatins' in the context of this invention means that the gelatinous polypeptides have a certain level of RGD motifs, calculated as a percentage of the total number of amino acids per molecule and a more even distribution of RGD motifs. RGD-enriched gelatins in the context of this invention are described in International Patent Applications WO 2004/085473 and WO 2008/103041. Preferably the recombinant gelatin comprises at least 3 RGD motifs and especially at least 5 RGD motifs per recombinant gelatin structure.

One or more different gelatins maybe cross-linked. Preferably a single gelatin is cross linked.

An important feature of the cross-linked hemostatic composition of the present invention is that the composition should be biodegradable and so not require invasive surgical methods for its removal.

*A priori* it is not obvious whether recombinant gelatins will be broken down by the same mechanisms causing degradation of natural gelatins. It is known that natural gelatins and collagens are degraded in the human body by proteases and more specifically matrix-metalloproteinases (MMP). Matrix metalloproteinases (MMP's) are zinc-dependent endopeptidases. The MMP's belong to a larger family of proteases known as the metzincin superfamily. Collectively they are capable of degrading all kinds of extracellular matrix proteins, but also can process a number of bioactive molecules. An important group of MMP's are the collagenases. These MMP's are capable of degrading triple-helical fibrillar collagens into distinctive 3/4 and 1/4 fragments. These collagens are the major components of bone and cartilage, and MMP's are the only known mammalian enzymes capable of degrading them. Traditionally, the collagenases are: MMP-1 (interstitial collagenase), MMP-8 (neutrophil collagenase), MMP-13 (collagenase 3) and MMP-18 (collagenase 4). Another important group of MMP's is formed by the gelatinases. The main substrates of these MMP's are type IV collagen and gelatin, and these enzymes are distinguished by the presence of an additional domain inserted into the catalytic domain. This gelatin-binding region is positioned immediately before the zinc binding motif, and forms a separate folding unit which does not disrupt the structure of the catalytic domain. The two members of this sub-group are: MMP-2 (72 kDa gelatinase, gelatinase-A) and MMP-9 (92 kDa gelatinase, gelatinase-B). However, International Patent Application WO/2008103045, incorporated herein by reference, discloses that a recombinant gelatin that does not comprise a known cleavage site for MMP was enzymatically degradable by human matrix metalloproteinase 1 (MMP1). Apparently many more types of recombinant gelatin than predicted can be degraded. Therefore the cross-linked recombinant gelatin will exhibit the required gradual biodegradation desirable for a hemostatic composition.

The preparation of the hemostatic composition of the current invention, can be performed by any method known in the art using any cross-linking agent that has a neutral effect on the surface charge, or that introduces positive charges in the cross-linked gelatin.

The cross-linking agents are carbodiimides such as but not limited to dicyclohexylcarbodiimide, N,N'-diisopropylcarbodiimide and 1-ethyl-3-[3-dimethylaminopropyl]carbodiimide. In a preferred embodiment the method for preparing the hemostatic composition of the current invention is performed by cross-linking the gelatin using a water soluble carbodiimide. In an even more preferred embodiment the gelatin is cross-linked using 1-ethyl-3-[3-dimethylaminopropyl]carbodiimide hydrochloride (EDC or EDAC). Cross-linking reaction conditions vary depending on which cross-linking agent is used and would be well known to a skilled person.

A preferred composition the according to the present invention comprises; 92 to 99.9 percent by weight of a cross-linked recombinant gelatin with an iso-electric point of at least 7.

Compositions according to the present invention may be prepared by any method which would be known to the skilled person.

Preferably compositions according to the present invention are prepared by a method which comprises:
(a) dissolving at least 0.1, 0.5, 1, 3, 6, 12, 24 to 35 weight volume percent of recombinant gelatin, in water;
(b) lyophilizing the solution;
(c) cross-linking the lyophilized material in a polar solvent, preferably ethanol, comprising a carbodiimide, preferably EDC at a concentration of at least 0.1, 0.25, 0.5, 1, 1.5, 2, 2.5 to 10 weight percent; and cross-linking for at least 10 minutes and up to 24 hours.

Compositions of the present invention may also preferably be prepared by a method which comprises:
(a) dissolving at least 0.1, 0.5, 1, 3, 6, 12, 24 to 35 weight volume percent of recombinant gelatin, in water;
(b) adjusting the pH of the solution (a) to between 3 and 6, more preferably to between 4 and 5 and even more preferably to a pH of 4.5;
(c) cross-linking the solution by adding EDC in an EDC/lysine residue, molar ratio of at least 0.6, 1.2, 3.6, 7.2, 12.5 for at least 10 minutes and up to 24 hours;
(d) ultraturaxing the cross-linked gelatin hydrogel and rinsing with water;
(e) lyophilizing the cross-linked gelatin hydrogel particles to obtain a powder.

A further aspect of the present invention provides the use of a composition comprising a cross-linked gelatin with an isoelectric point of at least 7 in the preparation of a medicament for use as a hemostatic agent. Preferences for the composition and all aspects of the gelatin and cross-linked gelatin are as described above.

The hemostatic agent of this invention is particularly applicable to the control of bleeding from surfaces, especially large surfaces. For example, the hemostatic agent is useful on (a) a cut or severed bone, (b) a severed organ, e.g. spleen, liver or kidney which has been cut surgically or traumatically, (c) the central nervous system where small blood vessels predominate, (d) prosthetic surgery, (e) oozing surfaces resulting from the surgical removal of necrotic tissue, (f) cosmetic surgery and (g) any surfaces with oozing of blood from one or more small sources, e.g. facial cuts.

The hemostatic agent may be applied in a variety of forms, e.g. as a powder directly to the surface; as a styptic in pencil form; as a gel, a sponge or in fabric form or as a coating on a surface of a medical device, wound dressing or other medical implement. The amount of agent employed varies with the extent of the bleeding surface and severity of the blood flow. Sufficient agent is applied to effect the desired control. Every application will have its own optimal degree of cross-linking.

The invention is illustrated in the following, non-limiting examples wherein all parts and percentages are by weight unless otherwise stated.

### Examples

Examples of the invention are illustrated in the accompanying figures.

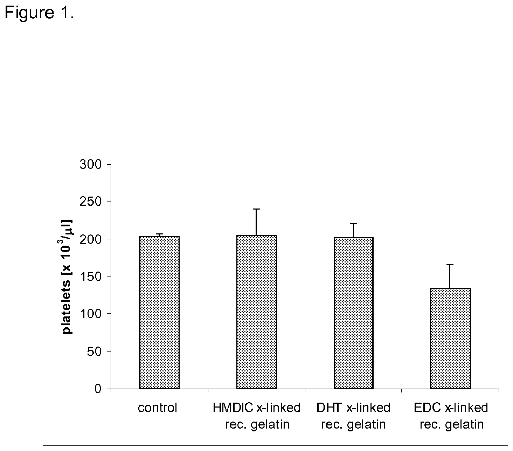

Figure 1 shows the effect of various cross-linked gelatins on the number of platelets in a blood sample;

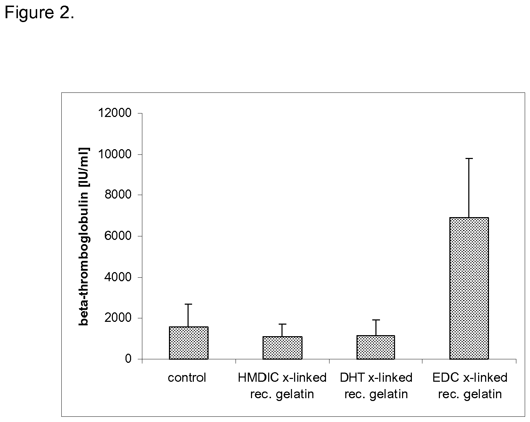

Figure 2 shows the level of β-thromboglobuline in a blood sample following contact with cross-linked gelatins;

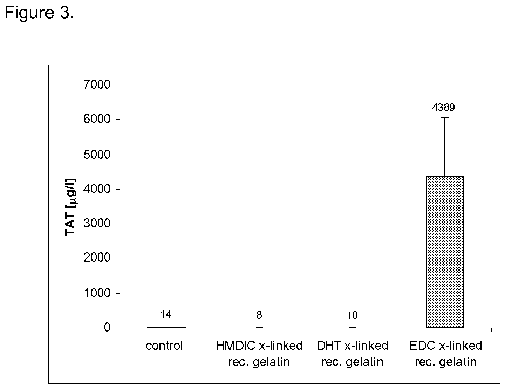

Figure 3 shows the formation of a thrombin-antithrombin complex in the presence of various cross-linked gelatins;

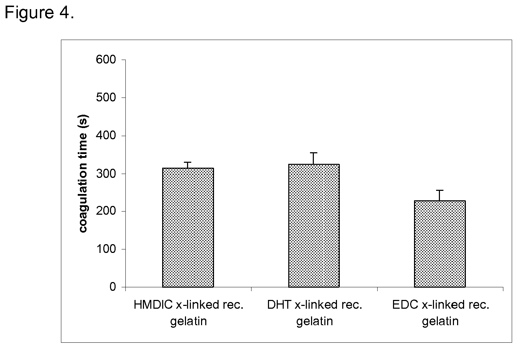

Figure 4 shows blood coagulation time in the presence of various cross-linked gelatins;

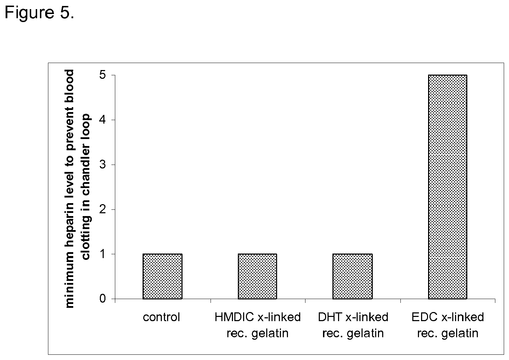

Figure 5 shows the minimum level of heparin required to prevent blood clotting in the presence of various cross-linked gelatins.

### Preparation Of A Hemostatic Agent Using Different Cross Linking Methods

Recombinant gelatin CBE3 prepared as described in International Patent Application WO2008103041 was used. The cross linking procedure was as follows: CBE3 solutions (1%) were buffered at pH 4 for EDC cross linking or buffered at pH 10 for hexamethylene diisocyanate (HMDIC) or dehydrothermal treatment cross-linking (DHT) and were then freeze dried. The freeze dried CBE3 solutions were cross-linked by reacting with EDC (0.25%) or HMDIC (0.0075%) dissolved in absolute ethanol for 24 hours. At the end of the reaction ethanol was removed by a vacuum drying at room temperature. DHT cross-linking was performed by an overnight heat-treatment of freeze dried CBE3 at 80°C followed by 8 hours at 160°C under vacuum conditions

### Assessment of Hemostatic Activity using the Dynamic Chandler loop method

The hemostatic effect of the various cross-linked gelatins was tested using the "Dynamic Chandler loop method" (Chandler AB et al. 1958, Lab Invest, 7: p110-114). This method uses hemostatic material inside a tube in a rotation mixer as a blood coagulation model. This model serves to investigate the ability of artificial surfaces to initiate the different cascade reactions of the human haemostatic system (coagulation, cell alteration, platelet and complement activation).

Blood was drawn from 3 healthy volunteers with an activated partial thromboplastin time in the normal range (age: greater than 20 and less than 40 years). The quality of the blood used for these experiments is of decisive importance. Therefore the following exclusion criteria for the blood donators were strictly fulfilled: no volunteers who smoked or had taken drugs in the previous 2 weeks, especially hemostasis-affecting agents like acetylsalicylic acid, oral contraceptives, non-steroidal antiphlogistics and others. Blood was drawn without stasis, very carefully, by venipuncture with "butterflies" (approximately, 1.4 mm) directly into sterile pre-anticoagulated containers. The anitcoagulant used was 5 IU/ml Heparin-Natrium-25000-ratiopharm (Ratiopharm GmbH, Ulm, Germany). All experiments used anticoagulant with the same lot No. Each experiment was performed in triplicate using 12.5 ml of blood from each of the three donors. The experiments were preformed at 37°C. The hemostatic agent sample added to the blood had a surface area of 50 cm². The control was blood in the absence of a cross linked gelatin.

Blood coagulation tests were carried out as follows:

| **Test category** | **Evaluation procedure** | **Determination** | **ELISA** | **Manufacturer** |
|---|---|---|---|---|
| 1. oagulation | Marker for Thrombin | Thrombin-Antithrombin III, complex (TAT) | ELISA | Dade Behring, Schwalbach, Germany |
| 2. Platelets | Number of platelets | Blood cell counting | Cell counter Micros 60 | ABX Hematology, Montpellier, France |
| | Marker for platelet activation | Beta-Thromboglobuline | ELISA | Diagnostica Stago/Roche, Mannheim, Germany |

### Results

### Figure 1

EDC cross-linked recombinant gelatin led to a reduction of blood platelets. Contact of blood with artificial surfaces leads to activation and alteration of platelets with consecutive loss of platelet functionality. A strong reduction in the amount of blood platelets is associated with a high thrombogenic potential. A significant reduction was observed in recombinant gelatin cross-linked with EDC. No reduction was observed when otherwise cross-linked.

### Figure 2

The dynamic Chandler loop experiments show that EDC cross-linked recombinant gelatin leads to activation of blood platelets. Activation of platelets by adhesion to a surface leads occurs in four steps: shape change with the formation of pseudopodia, adhesion, aggregation, and release of platelet factors out of the α-granules (platelet factor 4, ß-thromboglobulin, etc.). The concentration of ß-thromboglobulin in plasma corresponds with the degree of platelet activation.

### Figure 3

The dynamic Chandler loop experiments show that EDC cross-linked recombinant gelatin leads to the formation of a thrombin-antithrombin complex. Thrombin-antithrombin complex (TAT) is a sensitive marker for thrombin formation and therefore also an excellent marker for detection of coagulation activation. The TAT complex is formed after binding of generated thrombin with antithrombin-III. Extremely high TAT concentrations reflect a strong procoagulatory potency of the gelatins, particularly in an experiment with high heparin concentration (5 IU/ml).

### Clotting Kinetics

### Figure 4

Clotting kinetics were measured using a coagulometer (Biomatic 2000, Sarstedt), which monitors the progress of clotting optically by measuring the absorbance of a particular wavelength of light by the sample and how it changes over time. Blood was drawn from four healthy volunteers as described above. No heparin was added to these samples. A cross-linked gelatin hemostatic agent with a surface area of 50 cm² was added to the blood. The fastest coagulation time was detected in EDC cross-linked recombinant gelatin, indicating its strong procoagulatory potency.

### Example 5

Blood was drawn from donors as described in the dynamic Chandler loop experiments. Heparin-Natrium-25000-ratiopharm (Ratiopharm GmbH, Ulm, Germany) was added in a concentration of 1, 3 or 5 IU/ml to 12.5 ml blood. The blood was rotated for one hour at 37°C with 50 cm2 of hemostatic agent sample in a Chandler loop system, after which the minimum concentration of heparin to prevent macroscopically visible thrombus formation was determined. The concentration of heparin required to prevent the formation of macroscopically visible blood clots indicates the performance of the hemostyptic agents. A heparin concentration of 1 IU/ml was necessary for the amine-amine cross-linked gelatins, whereas EDC cross-linked recombinant gelatin still provoked blood clotting at this heparin concentration. Increasing the heparin concentration to 3 UI/ml still did not inhibit blood clotting with the EDC cross-linked recombinant gelatin. The addition of 5 UI/ml heparin was necessary to prevent thrombus formation in blood to which EDC cross-linked recombinant gelatin was added.

**Calculated and experimentally measured physiochemical properties of hemostatic agents prepared using different crosslinking agents.**

| | Non x-linked CBE3 | HMDIC x-linked CBE3 | DHT x-linked CBE3 | EDC x-linked CBE3 |
|---|---|---|---|---|
| NH2 amount per chain^{a} | 66 | 46 | 46 | 33 |
| COOH amount per chain^{b} | 58 | 58 | 58 | 25 |
| Nett charge/chain | +8 | -12 | -12 | +8 |
| IEP theoretically calculated after x-linking^{c} | 10.02* | 4.67 | 4.67 | 10.22 |
| Charge at neutral pH | +8.9 | -11 | -11 | +8.9 |
| Hemostatic performance | - | - | - | + |

| | | | | |
|---|---|---|---|---|
| ^{a} The number of free amine groups in uncross-linked and cross-linked CBE3 was chemically determined with a TNBS test (W.S. Hancock, J.E. Battersby, Anal. Biochem. 71 (1976) 260). ^{b} The number of free carboxyl groups was theoretically calculated, based on the known reaction chemistry of the cross-linker ^{c} The IEP was calculated assuming that all charged amino acids are equally available (no 3D structure, only linear chains) * determined before cross-linking | | | | |

## Claims

1. A composition comprising a cross-linked gelatin with an isoelectric point of at least 7, wherein the gelatin is a recombinant gelatin which comprises at least one RGD motif and the gelatin is cross-linked using a carbodiimide, for use as a hemostatic agent.

2. A composition for use as a hemostatic agent according to claim 1 wherein the gelatin which is to be cross-linked has an isoelectric point of at least 7 before cross-linking.

3. A composition for use as a hemostatic agent according to either claim 1 or claim 2 wherein the gelatin which is to be cross-linked has an isoelectric point of at least 9 before cross-linking.

4. A composition for use as a hemostatic agent according to any one of claims 1 to 3 wherein after cross-linking the cross-linked gelatin has an isoelectric point of at least 9.

5. A composition for use as a hemostatic agent according to claim 4 wherein the recombinant gelatin is free of hydroxyproline and hydroxylysine residues.

6. A composition for use as a hemostatic agent according to any one of the preceding claims wherein the recombinant gelatin comprises at least 0.40 mmol/g lysine residues before cross-linking.

7. A composition for use as a hemostatic agent according to any one of the preceding claims wherein the recombinant gelatin comprises a ratio between the total of arginine and lysine and the total of glutamine and aspargine aminoacid residues that is at least 1.0.

8. A composition for use as a hemostatic agent according to any one of the preceding claims wherein the recombinant gelatin has a molecular weight of at least 50 kDa.

9. A composition for use as a hemostatic agent according to any one of the preceding claims wherein the gelatin is cross-linked using 1-ethyl-3-[3-dimethylaminopropyl]carbodiimide hydrochloride (EDC).

10. A method for preparing the compositions for use as described in any one of claims 1 to 9 which comprises:
(a) dissolving at least 0.1 to 35 weight volume percent of gelatin in water;
(b) lyophilizing the solution;
(c) cross-linking the lyophilized material in a polar solvent comprising a carbodiimide at a concentration of at least 0.1 to 10 weight percent; and cross-linking for at least 10 minutes and up to 24 hours.

11. A method for preparing the compositions for use as described in any one of claims 1 to 9 which comprises:
(a) dissolving at least 0.1 to 35 weight volume percent of gelatin in water;
(b) adjusting the pH of the solution (a) to between 3 and 6;
(c) cross-linking the solution by adding EDC in an EDC/lysine residue, molar ratio of at least 0.6 for at least 10 minutes and up to 24 hours;
(d) ultraturaxing the cross-linked gelatin hydrogel and rinsing with water;
(e) lyophilizing the cross-linked gelatin hydrogel particles to obtain a powder.

## Patentansprüche

1. Zusammensetzung, umfassend eine vernetzte Gelatine mit einem isoelektrischen Punkt von mindestens 7, worin die Gelatine eine rekombinante Gelatine ist, die zumindest ein RGD-Motiv umfasst, und worin die Gelatine unter Verwendung eines Carbodiimids vernetzt ist, zur Verwendung als Hämostatikum.

2. Zusammensetzung zur Verwendung als Hämostatikum gemäß Anspruch 1, worin die zu vernetzende Gelatine einen isoelektrischen Punkt von mindestens 7 vor der Vernetzung aufweist.

3. Zusammensetzung zur Verwendung als Hämostatikum gemäß entweder Anspruch 1 oder Anspruch 2, worin die zu vernetzende Gelatine einen isoelektrischen Punkt von mindestens 9 vor der Vernetzung aufweist.

4. Zusammensetzung zur Verwendung als Hämostatikum gemäß irgendeinem der Ansprüche 1 bis 3, worin die vernetzte Gelatine nach der Vernetzung einen isoelektrischen Punkt von mindestens 9 aufweist.

5. Zusammensetzung zur Verwendung als Hämostatikum gemäß Anspruch 4, worin die rekombinante Gelatine keine Hydroxyprolin- und Hydroxylysin-Reste aufweist.

6. Zusammensetzung zur Verwendung als Hämostatikum gemäß irgendeinem der vorhergehenden Ansprüche, worin die rekombinante Gelatine mindestens 0,40 mmol/g Lysinreste vor der Vernetzung umfasst.

7. Zusammensetzung zur Verwendung als Hämostatikum gemäß irgendeinem der vorhergehenden Ansprüche, worin die rekombinante Gelatine ein Verhältnis zwischen der Gesamtmenge von Arginin- und Lysin- und der Gesamtmenge von Glutamin- und Asparagin-Aminosäureresten aufweist, das mindestens 1,0 beträgt.

8. Zusammensetzung zur Verwendung als Hämostatikum gemäß irgendeinem der vorhergehenden Ansprüche, worin die rekombinante Gelatine ein Molekulargewicht von mindestens 50 kDa aufweist.

9. Zusammensetzung zur Verwendung als Hämostatikum gemäß irgendeinem der vorhergehenden Ansprüche, worin die Gelatine unter Verwendung von 1-Ethyl-3-[3-dimethylaminopropyl]carbodiimid-Hydrochlorid (EDC) vernetzt ist.

10. Verfahren zur Herstellung der Zusammensetzungen zur Verwendung, wie sie in irgendeinem der Ansprüche 1 bis 9 beschrieben sind, welches umfasst:
(a) Auflösen von mindestens 0,1 bis 35 Gew.-Volumen% Gelatine in Wasser;
(b) Lyophilisieren der Lösung;
(c) Vernetzen des lyophilisierten Materials in einem polaren Lösungsmittel, das ein Carbodiimid in einer Konzentration von mindestens 0,1 bis 10 Gew.% umfasst; und Vernetzen für mindestens 10 Minuten und bis zu 24 Stunden.

11. Verfahren zur Herstellung der Zusammensetzungen zur Verwendung, wie in irgendeinem der Ansprüche 1 bis 9 beschrieben, welches umfasst:
(a) Auflösen von mindestens 0,1 bis 35 Gew.-Volumen% Gelatine in Wasser;
(b) Einstellen des pH-Werts der Lösung (a) auf zwischen 3 und 6;
(c) Vernetzen der Lösung durch Zugaben von EDC in einem EDC/Lysin-Rest, wobei das molare Verhältnis mindestens 0,6 beträgt, für mindestens 10 Minuten und bis zu 24 Stunden;
(d) Ultraturaxieren des vernetzten Gelatine-Hydrogels und Waschen mit Wasser;
(e) Lyophilisieren der vernetzten Gelatine-Hydrogelpartikel, um ein Pulver zu erhalten.

## Revendications

1. Composition comprenant une gélatine réticulée ayant un point isoélectrique d'au moins 7, dans laquelle la gélatine est une gélatine recombinante comprenant au moins un motif RGD, et la gélatine est réticulée par l'utilisation d'un carbodiimide, destinée à être utilisée en tant qu'agent hémostatique.

2. Composition destinée à être utilisée en tant qu'agent hémostatique selon la revendication 1, dans laquelle la gélatine qui doit être réticulée présente un point isoélectrique d'au moins 7 préalablement à la réticulation.

3. Composition destinée à être utilisée en tant qu'agent hémostatique selon la revendication 1 ou la revendication 2, dans laquelle la gélatine qui doit être réticulée présente un point isoélectrique d'au moins 9 préalablement à la réticulation.

4. Composition destinée à être utilisée en tant qu'agent hémostatique selon l'une quelconque des revendications 1 à 3, dans laquelle, après réticulation, la gélatine réticulée présente un point isoélectrique d'au moins 9.

5. Composition destinée à être utilisée en tant qu'agent hémostatique selon la revendication 4, dans laquelle la gélatine recombinante est exempte de résidus d'hydroxyproline et d'hydroxylysine.

6. Composition destinée à être utilisée en tant qu'agent hémostatique selon l'une quelconque des revendications précédentes, dans laquelle la gélatine recombinante comprend au moins 0,40 mmol/g de résidus de lysine avant la réticulation.

7. Composition destinée à être utilisée en tant qu'agent hémostatique selon l'une quelconque des revendications précédentes, dans laquelle la gélatine recombinante comprend un rapport entre le total des résidus d'aminoacides arginine et lysine et le total des résidus d'aminoacides glutamine et aspargine qui est d'au moins 1,0.

8. Composition destinée à être utilisée en tant qu'agent hémostatique selon l'une quelconque des revendications précédentes, dans laquelle la gélatine recombinante a un poids moléculaire d'au moins 50 kDa.

9. Composition destinée à être utilisée en tant qu'agent hémostatique selon l'une quelconque des revendications précédentes, dans laquelle la gélatine est réticulée par l'utilisation d'hydrochlorure de 1-éthyl-3-[3-diméthylaminopropyl]carbodiimide (EDC).

10. Procédé de préparation des compositions destinées à être utilisées comme décrit dans l'une quelconque des revendications 1 à 9, comprenant les étapes suivantes consistant à :
- (a) dissoudre au moins de 0,1 à 35 pourcent en poids/volume de gélatine dans de l'eau ;
- (b) lyophiliser la solution ;
- (c) réticuler la matière lyophilisée dans un solvant polaire comprenant un carbodiimide à une concentration d'au moins 0,1 à 10 pourcent en poids; et réticuler pendant au moins 10 minutes et jusqu'à 24 heures.

11. Procédé de préparation des compositions destinées à être utilisées comme décrit dans l'une quelconque des revendications 1 à 9, comprenant les étapes suivantes consistant à :
(a) dissoudre au moins de 0,1 à 35 pourcent en poids/volume de gélatine dans de l'eau ;
(b) ajuster le pH de la solution (a) entre 3 et 6 ;
(c) réticuler la solution par ajout de EDC dans un résidu EDC/lysine, de rapport molaire d'au moins 0,6 pendant au moins 10 minutes et jusqu'à 24 heures;
(d) disperser / homogénéiser par un dispositif Ultra-Turrax® l'hydrogel de gélatine réticulée et rincer avec de l'eau ;
(e) lyophiliser les particules d'hydrogel de gélatine réticulée afin d'obtenir une poudre.
